# EUROPÄISCHE PATENTSCHRIFT

(11) **EP 1 529 836 B1**
(45) Veröffentlichungstag und Bekanntmachung des Hinweises auf die Patenterteilung: **09.04.2008**
(21) Anmeldenummer: 04025370.0
(22) Anmeldetag: 26.10.2004
(51) Int. Cl.: C12M 3/00

(54) **Zellbehandlungskammer**
Cell treating chamber
Chambre de traitement de cellules

(30) Priorität: 06.11.2003 DE 10351833
(43) Veröffentlichungstag der Anmeldung: 11.05.2005
(73) Patentinhaber: EPPENDORF AG, 22339 Hamburg (DE)
(72) Erfinder: Huhn, Rüdiger, 23560 Lübeck (DE)
(74) Vertreter: Hauck Patent- und Rechtsanwälte

(56) Entgegenhaltungen:
- DE-A- 19 917 571
- DE-U- 8 813 314
- US-A- 4 764 473

## Beschreibung

Die Erfindung bezieht sich auf eine Kammer zum Behandeln von in einer Suspension enthaltenen Zellen im elektrischen Feld.

Die erfindungsgemäße Kammer dient z.B. der Elektrofusion von Zellen. Bei der Elektrofusion werden zumindest zwei Zellen in einem elektrischen Feld fusioniert, das zwischen zwei Elektroden aufgebaut wird. Die Elektrofusion kann z.B. in drei Schritten verlaufen. In einem ersten Schritt (pre-alignment) werden die Zellen durch Anlegen einer hochfrequenten Wechselspannung ausgerichtet und miteinander in Kontakt gebracht. In einem zweiten Schritt (pulse) werden mittels eines kurzen, starken Strompulses die Zellmembranen aufgebrochen. In einem dritten Schritt (post-alignment) werden die aufgebrochenen Zellen durch erneutes Anlegen einer hochfrequenten Wechselspannung verschmolzen bzw. gehalten, um ein selbsttätiges Verschmelzen zu ermöglichen.

Bekannt sind Zellfusionskammern, bei denen in ein Paar schraubenlinienförmiger Rillen auf einem konischen Körper aus nichtleitendem Kunststoff zwei dünne Platindrähte parallel aufgewickelt sind, die die Elektroden bilden. Ferner haben die Zellfusionskammern einen konischen Becher, in den der Kunststoffkörper einschraubbar ist. Der Kunststoffkörper hat einen über die Oberseite des Bechers hinausstehenden Kopf, an dem ein Stecker montiert ist, der mit den Elektroden verbunden ist. Über den Stecker ist die hochfrequente Wechselspannung zuführbar. Die Herstellung der Zellfusionskammern ist aufwendig und teuer, so daß eine mehrfache Nutzung der Zellfusionskammern wirtschaftlich wünschenswert ist. Hierfür müssen die Zellfusionskammern zwischen verschiedenen Anwendungen aufwendig mechanisch und mit Reinigungsmitteln gereinigt werden. Die bekannten Zellfusionskammern sind nicht autoklavierbar bzw. sterilisierbar, da sie nach einer entsprechenden Behandlung meist nicht mehr funktionsfähig sind.

Die US-A-4 764 473 offenbart eine Zellbehandlungskammer mit zwei dünnen Platindrähten auf einem zylindrischen Körper aus Polymethacrylat (PMMA). Diese Zellfusionskammer ist nach dem Autoklavieren oder Sterilisieren meist nicht mehr funktionsfähig.

Davon ausgehend liegt der Erfindung die Aufgabe zugrunde, eine Zellfusionskammer zu schaffen, die autoklavierbar und/oder sterilisierbar ist.

Die Aufgabe wird durch eine Kammer mit den Merkmalen des Anspruches 1 gelöst. Vorteilhafte Ausgestaltungen der Kammer sind in den Unteransprüchen angegeben.

Die erfindungsgemäße Kammer zum Behandeln von in einer Suspension enthaltenen Zellen im elektrischen Feld hat
- einen Becher aus elektrisch nichtleitendem Material, in den durch eine Öffnung axial ein länglicher Kern aus elektrisch nichtleitendem Material mindestens teilweise eingesetzt ist, wobei zwischen Becher und Kern ein Raum zur Aufnahme der Suspension vorhanden ist,
- mindestens zwei auf der dem Raum zugewandten Außenseite des Kerns angeordnete Elektroden aus elektrisch leitendem Material, zwischen denen durch Anlegen einer Spannung ein elektrisches Feld zum Behandeln von Zellen in einer in dem Raum enthaltenen Suspension erzeugbar ist,
- wobei die thermischen Ausdehnungskoeffizienten des Materials der Elektroden und des Materials des Kerns so einander angeglichen sind, daß die Elektroden im Temperaturbereich von Umgebungstemperatur bis hin zu den beim Autoklavieren und/oder Sterilisieren erreichten Temperaturen ihre Lage bezüglich des Kerns im wesentlichen beibehalten.

Im Rahmen der Erfindung wurde erkannt, daß die mangelnde Autoklavierbarkeit und Sterilisierbarkeit herkömmlicher Zellfusionskammern darauf beruht, daß die Platindrähte infolge Erwärmung ihre Lage auf dem Kern verändern. Es wurde erkannt, daß dies auf der stark unterschiedlichen thermischen Ausdehnung der Platindrähte und des Kunststoffmaterials des Kerns beruht. Der um gut eine Zehnerpotenz größere thermische Ausdehnungskoeffizient (Anmerkung: maßgeblich ist der thermische Längenausdehnungskoeffizient) des Kernmaterials führt dazu, daß sich bei einer Temperaturerhöhung der Kern stärker ausdehnt als die Drähte. Der Draht wird dabei plastisch verformend gedehnt. Bei Temperaturabsenkung verrutschen dann die Drähte bezüglich des Kerns, so daß es zu Kurzschlüssen zwischen den verschiedenen Windungen kommt. Erfindungsgemäß wird dies durch Angleichung der thermischen Ausdehnungskoeffizienten des Materials der Elektroden und des Materials des Kerns vermieden. Die Angleichung ist soweit erfolgt, daß die Elektroden ihre Lage bezüglich des Kerns im gesamten Temperaturbereich von Umgebungstempertur (etwa -21 bis +35 °C) bis zu den typischen Temperaturen beim Autoklavieren und/oder Sterilisieren (etwa +120 bis +180 °C) im wesentlichen beibehalten. Die Elektroden behalten ihre Lage bezüglich des Kerns im wesentlichen bei, wenn sie sich nicht so weit verlagern, daß ein Kurzschluß eintritt.

Gemäß einer Ausgestaltung sind die thermischen Ausdehnungskoeffizienten des Materials der Elektroden und des Materials des Kerns so einander angeglichen, daß die Elektroden bis hin zu den bei Dampfsterilisation und/oder Strahlensterilisation erreichten Temperaturen (etwa +120 bis +140 °C) ihre Lage bezüglich des Kerns nicht wesentlich ändern.

Gemäß einer Ausgestaltung ist der Kern zylindrisch oder konisch und/oder der Becher außen und/oder innen zylindrisch und/oder konisch. Hierdurch wird insbesondere das Herstellen der Elektroden durch Aufwickeln von Drähten begünstigt. Bei komplementären Formen von Kern und Becher hat der Raum eine gleichmäßige Tiefe, was wiederum vorteilhaft für die gleichmäßige Behandlung der Zellen in der Suspension ist.

Gemäß einer Ausgestaltung sind die Elektroden Drähte, die mehrgängig schraubenlinienförmig oder rampenförmig auf den Kern gewickelt sind. Der schraubenlinienförmige Verlauf ist z.B. bei einem zylindrischen oder konischen und der rampenförmige Verlauf z.B. bei einem polyedrischen Kern gegeben.

Gemäß einer Ausgestaltung hat der Kern mehrere schraubenlinienförmig oder rampenförmig verlaufende Rillen oder Nuten an der Außenseite, in denen die Drähte geführt sind. Hierdurch wird zusätzlich ein Verrutschen der Drähte verhindert, das infolge geringer verbleibender Unterschiede des thermischen Ausdehnungskoeffizienten oder aufgrund mechanischer Einwirkung auf die Drähte eintreten könnte.

Gemäß einer Ausgestaltung weisen die Drähte abgewinkelte Enden auf, die in Radialbohrungen des Kerns eingesetzt sind. Hierdurch werden die Drähte sicher auf dem Kern fixiert. Durch die Radialbohrungen sind zudem die Drähte mit einem HF-Spannungsgenerator verbindbar. Gemäß einer weiteren Ausgestaltung sind die abgewinkelten Drahtenden mittels eines nicht zytotoxischen Klebstoffes in den Radialbohrungen fixiert. Es versteht sich, daß der Klebstoff in dem Temperaturbereich, in dem die Kammer verwendbar ist, beständig ist.

Gemäß einer Ausgestaltung sind die Elektroden auf dem Kern angeordnete Leiterbahnen. Die Leiterbahnen sind z.B. durch flächige Beschichtung des Kerns mit einem elektrisch leitenden Material, partielles Abdecken der als Leiterbahnen vorgesehenen Bereiche und chemisches Ablösen der nicht abgedeckten Bereiche der Beschichtung herstellbar. Gemäß einer Ausgestaltung ist zwischen dem Kern und den Leiterbahnen ein Haftvermittler angeordnet, der die Haftung des Materials der Leiterbahnen an dem Material des Kerns verstärkt.

Die Leiterbahnen verlaufen z.B. ebenfalls mehrgängig schraubenlinienförmig oder parallel zur Längsachse des Kerns. Gemäß einer Ausgestaltung weisen die Leiterbahnen eine Interdigitalstruktur auf, d.h. kammartige, ineinandergreifende Bereiche.

Der Kern ist auf verschiedene Weise bezüglich des Bechers fixierbar. Vorzugsweise erfolgt eine Fixierung direkt am Becher. Hierzu weist gemäß einer Ausgestaltung der Kern oberhalb der Elektroden ein Außengewinde und der Becher unterhalb einer Öffnung ein Innengewinde zum Einschrauben des Außengewindes auf. Der Kern ist dann einfach am Ende einer axialen Einsetzbewegung in den Becher einschraubbar. Gemäß einer Ausgestaltung weist der Kern oberhalb der Elektroden einen Kopf auf. Der Kopf ist zur Handhabung des Kerns nutzbar, wodurch eine Kontamination der Elektroden vermeidbar ist. Bevorzugt der Kopf am Umfang in einer das Einschrauben in den Becher fördernden Weise geformt.

Gemäß einer Ausgestaltung hat der Kern eine von der Oberseite ausgehende Sackbohrung, in die die Elektroden hineingeführt sind. Die Elektroden sind durch die Sackbohrung von außen kontaktierbar, um die HF-Spannung anzulegen.

Gemäß einer weiteren, das Kontaktieren erleichternden Ausgestaltung sind die Elektroden mit einem Stecker an der Oberseite des Kerns verbunden.

Gemäß einer Ausgestaltung sind die Elektroden aus Platin. Als inertes Material ist Platin besonders für die Behandlung von Zellen in einer Suspension geeignet. Sowohl als Drähte als auch als Leiterbahnen ausgeführte Elektroden sind aus Platin herstellbar. Platin hat einen thermischen Ausdehnungskoeffizienten von 9,0 x 10⁻⁶ 1/K.

Gemäß einer Ausgestaltung ist der Kern aus Glaskeramik. So steht beispielsweise eine Glaskeramik mit einem thermischen Ausdehnungskoeffizienten von 9,3 x 10⁻⁶ 1/K zur Verfügung. Besonders vorteilhaft ist die Kombination von Elektroden aus Platin mit einem Kern aus Glaskeramik, da diese das inerte Material der Elektroden mit einer vollständigen Angleichung der thermischen Ausdehnungskoeffizienten verbindet. Bei dieser Materialkombination wird erreicht, daß die Elektroden ihre Position bezüglich des Kerns im gesamten Temperaturbereich von Umgebungstemperatur bis hin zu Temperaturen beim Autoklavieren und/oder Sterilisieren im wesentlichen beibehalten. Platin und Glaskeramik sind überdies strahlensterilisierbar.

Gemäß einer Ausgestaltung ist der Kern aus spanbarer Glaskeramik spanend geformt oder aus Glaskeramik spritzgegossen. Ein geeignetes spanbares Material wird unter der Produktbezeichnung MACOR^{®} von der Firma Corning angeboten. Beim Keramikspritzgußverfahren wird Keramik im Pulverzustand mit polymerer Matrix gemischt und zu einem Granulat verarbeitet. Damit wird eine Spritzgußmaschine gespeist, die einen Grünling liefert. Dieser wird gesintert, um die organische Phase im wesentlichen auszutreiben und die Keramikartikel zusammenzubacken.

Bei der bekannten Kammer bestehen Kern und Becher aus PMMA und der Stecker aus PTFE. Diese Materialien haben den zusätzlichen Nachteil, nicht strahlensterilisierbar zu sein. Gemäß einer Ausgestaltung besteht der Becher aus PC und/oder der Stecker aus PEEK. Diese Materialien sind strahlensterilisierbar.

Die Erfindung wird nachfolgend anhand der anliegenden Zeichnungen von Ausfiihrungsbeispielen näher erläutert. In den Zeichnungen zeigen:
- Fig. 1: einen Kern in der Seitenansicht;
- Fig. 2: ein vergrößertes Detail II von Fig. 1;
- Fig. 3: den Kern in der Draufsicht;
- Fig. 4: einen oberen Abschnitt des Kerns in einem Längsschnitt;
- Fig. 5: die Kammer in einem Längsschnitt;
- Fig. 6: eine weitere Kammer in einem Längsschnitt.

Mit "oben" und "unten" ist die Lage von Elementen der Kammer in einer typischen Anwendungssituation bezeichnet, in der der Becher mit dem Boden auf einem Untergrund plaziert und mit der Öffnung vertikal über dem Boden angeordnet ist. Dementsprechend ist der koaxial zum Becher ausgerichtete Kern mit dem Kopf und dem Stecker vertikal über der Öffnung des Bechers angeordnet.

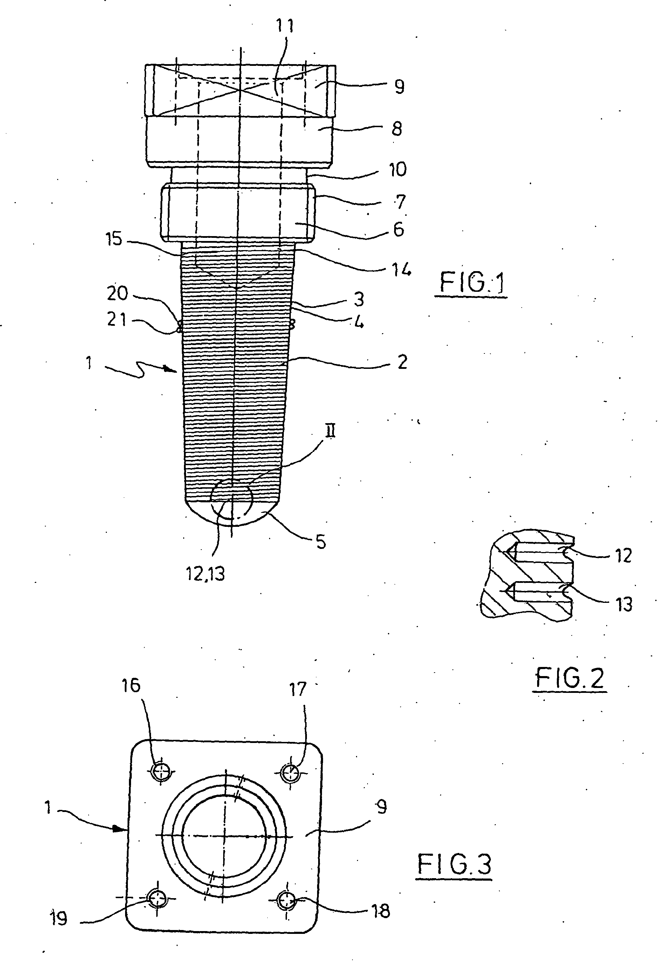

Gemäß Fig. 1 bis 4 hat ein Kern 1 einen leicht konischen Tragabschnitt 2, auf dem zwei parallele Rillen 3, 4 schraubenlinienförmig verlaufen. Am Ende des Tragabschnittes 2 mit dem geringeren Durchmesser weist der Kern 1 einen balligen unteren Endabschnitt 5 auf. Am Ende des Tragabschnittes 2 mit dem größeren Durchmesser hat der Kern 1 einen Gewindeabschnitt 6, der einen deutlich größeren Durchmesser als das angrenzende Ende des Abschnittes 2 und ein Außengewinde 7 aufweist.

Oben weist der Kern 1 einen Kopf 8 auf, der im oberen Bereich zu einer rechteckigen Platte 9 ausgeformt ist. Kopf 8 und Gewindeabschnitt 7 sind durch einen Einschnitt 10 voneinander getrennt.

Ferner hat der Kern 1 eine vom oberen Ende ausgehende Sackbohrung 11, die in Längsrichtung des Kerns 1 bis in den Bereich des Tragabschnittes 2 verläuft. Zudem hat der Tragabschnitt 2 am unteren Ende zwei übereinander angeordnete Radialbohrungen 12, 13, die als Sackbohrungen ausgeführt sind. Am oberen Ende hat er zwei diametral einander gegenüberliegende Radialbohrungen 14, 15, die in der Sackbohrung 11 münden. In der Platte 9 hat der Kern 1 vier kurze axiale Gewindebohrungen 16, 17, 18, 19.

Elektroden in Form von Drähten aus Platin 20, 21 sind parallel auf den Tragabschnitt 2 aufgewickelt und mit ihren Enden in die Radialbohrungen 12, 13 und 14, 15 hineingeführt. In den Radialbohrungen sind sie mittels eines geeigneten, nicht zytotoxischen Klebstoffes fixiert. In der Zeichnung sind nur zwei parallele Windungen der Platindrähte 20, 21 eingetragen. Die Platindrähte 20, 21 erstrecken sich jedoch tatsächlich im wesentlichen über die gesamte Länge der Rillen 3, 4.

Ein - nicht gezeigter - Stecker ist am Kopf 8 des Kerns 1 angeordnet und dort mit den Gewindebohrungen 16, 17, 18, 19 der Platte 9 verschraubt. Der Stecker ist mit den oberen Enden der Drähte 20, 21 elektrisch verbunden.

Die Elektroden 20, 21 bestehen also aus Platindraht. Der Kern 1 ist aus MACOR^{®} Glaskeramik der Firma Corning spanend hergestellt. Der Stecker ist aus PEEK hergestellt.

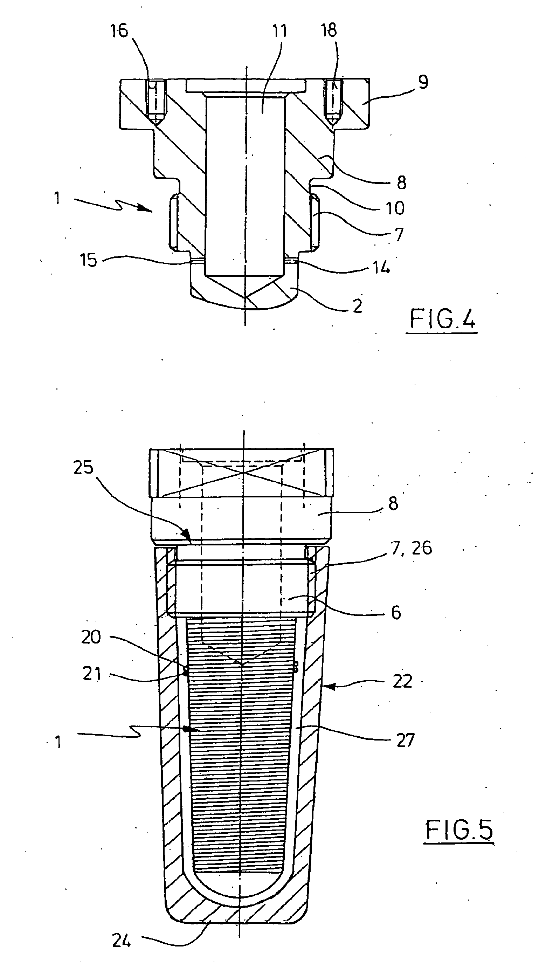

Gemäß Fig. 5 hat ein zugehöriger Becher 22 einen innen und außen konischen Mantel 23, dessen Konuswinkel dem des Tragabschnittes 2 des Kerns 1 entspricht. Ferner hat der Becher 22 einen Boden 24 mit einer flachen Unterseite und diesem gegenüber am oberen Ende eine Öffnung 25. Unterhalb der Öffnung 25 ist ein Innengewinde 26 vorhanden. Der Becher 22 besteht aus PC.

Für die Behandlung von in einer Suspension enthaltenen Zellen werden zunächst Kern 1 und Becher 22 autoklaviert oder dampf- bzw. strahlensterilisiert. Aufgrund der eingesetzten Materialien ist dies ohne Beeinträchtigung der Funktionsfähigkeit der Kammer 1, 22 möglich.

Dann wird die Suspension in den Becher 22 eingefüllt und schließlich der Kern 1 axial eingeführt und eingeschraubt. Die Suspension füllt dann den Raum 27 zwischen Kern 1 und Becher 22 aus.

Schließlich wird ein HF-Spannungsgenerator an den Stecker angeschlossen und die Behandlung der Zellen durchgeführt.

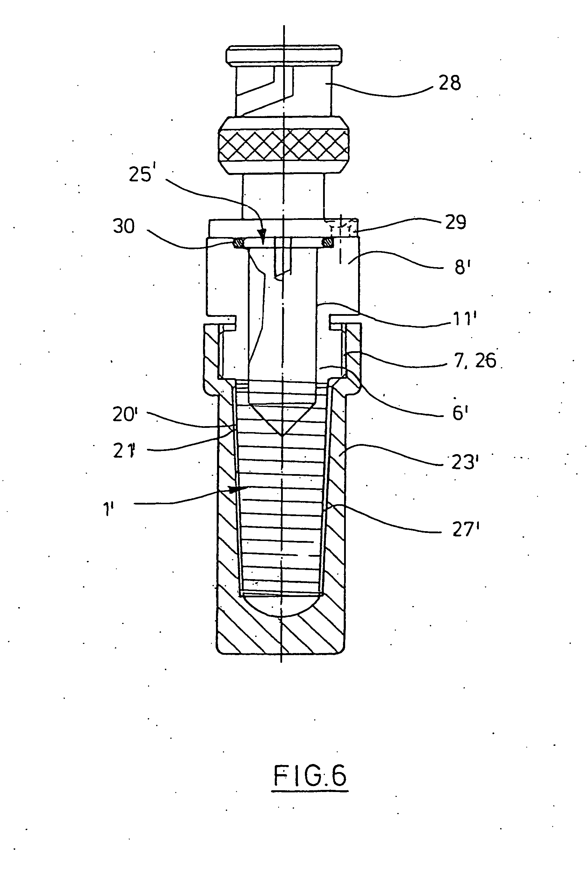

Bei dem Ausführungsbeispiel von Fig. 6 sind die mit dem Ausführungsbeispiel von Fig. 1 bis 5 im wesentlichen übereinstimmenden Merkmale mit denselben Bezugsziffern bezeichnet, das jedoch mit einem Anstrich versehen ist.

Bei der Kammer von Fig. 6 weist der Kern 1' einen Stecker 28 auf, der oben am Kopf 8' fixiert ist. Hierfür hat der Stecker 28 eine Befestigungsplatte 29, die mit der Oberseite des Kopfes 8' verschraubt ist, wobei ein Dichtungsring 30 zwischengelegt ist. Die Platindrähte 20', 21' sind innerhalb der Sackbohrung 11' mit Kontakten des Steckers 28 verbunden.

Der Becher 23' ist innen konisch entsprechend dem Konus des Kerns 1'. Außen ist der Becher 23' zylindrisch.

Im übrigen gelten die Erläuterungen zum Ausführungsbeispiel der Fig. 1 bis 5.

## Patentansprüche

1. Kammer zum Behandeln von in einer Suspension enthaltenen Zellen im elektrischen Feld mit
- einem Becher (22) aus elektrisch nichtleitendem Material, in den durch eine Öffnung (25) axial ein länglicher Kern (1) aus elektrisch nichtleitendem Material mindestens teilweise eingesetzt ist, wobei zwischen Becher (22) und Kern (1) ein Raum (27) zur Aufnahme der Suspension vorhanden ist,
- mindestens zwei auf der dem Raum (27) zugewandten Außenseite des Kerns (1) angeordneten Elektroden (20, 21) aus elektrisch leitendem Material, zwischen denen durch Anlegen einer Spannung ein elektrisches Feld zum Behandeln von Zellen in einer in dem Raum (27) enthaltenen Suspension erzeugbar ist,
- wobei die thermischen Ausdehnungskoeffizienten des Materials der Elektroden (20, 21) und des Materials des Kerns (1) so einander angeglichen sind, daß die Elektroden (20, 21) ihre Position bezüglich des Kerns im Temperaturbereich von Umgebungstemperatur bis hin zu den beim Autoklavieren und/oder Sterilisieren erreichten Temperaturen nicht wesentlich verändern.

2. Kammer nach Anspruch 1, bei der die thermischen Ausdehnungskoeffizienten des Materials der Elektroden (20, 21) und des Materials des Kerns (1) so aneinander angeglichen sind, daß die Elektroden (20, 21) ihre Position bezüglich des Kerns (1) bis zu den beim Dampf- und/oder Strahlensterilisieren erreichten Temperaturen nicht wesentlich verändern.

3. Kammer nach Anspruch 1 oder 2, bei der der Kern (1) zylindrisch oder konisch und/oder der Becher (22) außen und/oder innen zylindrisch und/oder konisch ist.

4. Kammer nach einem der Ansprüche 1 bis 3, bei der die Elektroden (20, 21) Drähte sind, die mehrgängig schraubenlinienförmig oder rampenförmig auf den Kern (1) gewickelt sind.

5. Kammer nach Anspruch 4, bei der der Kern (1) mehrere parallele schraubenlinienförmig oder rampenförmig verlaufende Rillen (3, 4) oder Nuten an der Außenseite hat, in denen die Drähte (20, 21) geführt sind.

6. Kammer nach Anspruch 4 oder 5, bei der die Drähte (20, 21) abgewinkelte Enden aufweisen, die in Radialbohrungen (12, 13, 14, 15,) des Kerns (1) eingesetzt sind.

7. Kammer nach Anspruch 6, bei der die abgewinkelten Enden mittels eines nicht zytotoxischen Klebstoffs in den Radialbohrung (12, 13, 14, 15) fixiert ist.

8. Kammer nach einem der Ansprüche 1 bis 3, bei der die Elektroden (20, 21) auf dem Kern (1) angeordnete Leiterbahnen sind.

9. Kammer nach Anspruch 8, bei der zwischen dem Kern (1) und den Leiterbahnen ein Haftvermittler angeordnet ist.

10. Kammer nach einem der Ansprüche 1 oder 9, bei der die Leiterbahnen eine Interdigitalstruktur aufweisen.

11. Kammer nach einem der Ansprüche 1 bis 10, bei der der Kern (1) oberhalb der Elektroden (20, 21) ein Außengewinde (7) und der Becher (22) unterhalb einer Öffnung (25) ein Innengewinde (26) zum Einschrauben des Außengewindes (7) aufweist.

12. Kammer nach einem der Ansprüche 1 bis 11, bei der der Kern (1) oberhalb der Elektroden (20, 21) einen Kopf (8) aufweist.

13. Kammer nach einem der Ansprüche 1 bis 12, die eine axiale Sackbohrung (11) aufweist, die vom oberen Ende des Kerns (1) aus bis etwa auf das Niveau der Elektroden (20, 21) erstreckt ist.

14. Kammer nach einem der Ansprüche 1 bis 13, bei der die Elektroden (20, 21) mit einem Stecker (28) an der Oberseite des Kerns (1) verbunden sind.

15. Kammer nach einem der Ansprüche 1 bis 14, bei der die Elektroden (20, 21) aus Platin sind.

16. Kammer nach einem der Ansprüche 1 bis 15, bei der der Kern (1) aus Glaskeramik ist.

17. Kammer nach einem der Ansprüche 1 bis 16 mit einem aus spanbarer Glaskeramik spanend geformten oder aus Glaskeramik spritzgegossenen Kern (1).

18. Kammer nach einem der Ansprüche 1 bis 17 mit einem Becher (22) aus PC.

19. Kammer nach einem der Ansprüche 14 bis 18 mit einem Stecker (28) aus PEEK.

## Claims

1. Chamber for treating cells contained in a suspension in the electrical field with
- a beaker (22) made from electrically non-conductive material, into which an elongate core (1) made from electrically non-conductive material is at least partially inserted axially through an opening (25), between the beaker (22) and the core (1) a gap (27) being present to receive the suspension.
- at least two electrodes (20, 21) made from electrically conductive material arranged on the outer face of the core (1) facing the gap (27), between which an electrical field can be created to treat cells in a suspension contained in the gap (27) by applying a voltage,
- whereas the thermal expansion coefficient of the material of the electrodes (20, 21) and of the material of the core (1) being matched with one another, such that the electrodes (20, 21) do not substantially alter their position relative to the core in the temperature range from ambient temperature up to temperatures reached during autoclaving and/or sterilising.

2. Chamber according to claim 1, in which the thermal expansion coefficient of the material of the electrodes (20, 21) and the material of the core (1) are matched with one another such that the electrodes (20, 21) do not substantially alter their position relative to the core (1) up to temperatures reached during steam- and/or radiation sterilisation.

3. Chamber according to claim 1 or 2, in which the core (1) is cylindrical or conical and/or the beaker (22) is cylindrical and/or conical externally and/or internally.

4. Chamber according to any of claims 1 to 3, in which the electrodes (20, 21) are wires, which are wound as a helical- or ramp-shaped multi-thread on the core (1).

5. Chamber according to claim 4, in which the core (1) has a plurality of helically or ramp-shaped extending flutes (3, 4) or grooves on the outer face in which the wires (20, 21) are guided.

6. Chamber according to claim 4 or 5, in which the wires (20, 21) comprise offset ends which are inserted into radial bores (12, 13, 14, 15) of the core (1).

7. Chamber according to claim 6, in which the offset ends are fixed by means of a non-cytotoxic adhesive in the radial bores (12, 13, 14, 15).

8. Chamber according to any of claims 1 to 3, in which the electrodes (20, 21) are conductor paths arranged on the core (1).

9. Chamber according to claim 8, in which a bonding agent is arranged between the core (1) and the conductor paths.

10. Chamber according to either claim 1 or 9, in which the conductor paths comprise an interdigital structure.

11. Chamber according to any of claims 1 to 10, in which the core (1) comprises a male thread (7) above the electrodes (20, 21) and the beaker (22) comprises a female thread (26) below an opening (25) for screwing in the male thread (7).

12. Chamber according to any of claims 1 to 11, in which the core (1) comprises a head (8) above the electrodes (20, 21).

13. Chamber according to any of claims 1 to 12, which comprise an axial blind hole (11) which is extended from the upper end of the core (1) as far as approximately level with the electrodes (20, 21).

14. Chamber according to any of claims 1 to 13, in which the electrodes (20, 21) are connected to a plug (28) on the upper face of the core (1).

15. Chamber according to any of claims 1 to 14, in which the electrodes (20, 21) are made from platinum.

16. Chamber according to any of claims 1 to 15, in which the core (1) is made from glass ceramic.

17. Chamber according to any of claims 1 to 16 with a core (1) machined from machinable glass ceramic or injection moulded from glass ceramic.

18. Chamber according to any of claims 1 to 17 with a beaker (22) made from PC.

19. Chamber according to any of claims 14 to 18 with a plug (28) made from PEEK.

## Revendications

1. Chambre de traitement de cellules contenues dans une suspension, dans un champ électrique, comportant
- un bécher (22) en matériau non électroconducteur, dans lequel un noyau (1) allongé en matériau non électroconducteur est introduit au moins partiellement dans le sens axial à travers une ouverture (25), un espace (27) pour la réception de la suspension étant formé entre le bécher (22) et le noyau (1),
- au moins deux électrodes (20, 21) en matériau électroconducteur, qui sont disposées sur la face extérieure du noyau (1), orientée vers l'espace (27), entre lesquelles, un champ électrique pour le traitement de cellules dans une suspension contenue dans l'espace (27) peut être généré, moyennant l'application d'une tension,
- les coefficients de dilatation thermique du matériau des électrodes (20, 21) et du matériau du noyau (1) étant adaptés entre eux de telle sorte que les électrodes (20, 21) ne changent pas nettement leur position par rapport au noyau dans le domaine des températures allant de la température ambiante jusqu'à des températures atteintes lors du traitement en autoclave et/ou lors de la stérilisation.

2. Chambre selon la revendication 1, dans laquelle les coefficients de dilatation thermique du matériau des électrodes (20, 21) et du matériau du noyau (1) sont adaptés entre eux de telle sorte que les électrodes (20, 21) ne changent pas nettement leur position par rapport au noyau (1) jusqu'à des températures atteintes lors de la stérilisation par vapeur et/ou par rayonnement.

3. Chambre selon la revendication 1 ou 2, dans laquelle le noyau (1) est cylindrique ou conique et/ou le bécher (22) est cylindrique et/ou conique à l'extérieur et/ou à l'intérieur.

4. Chambre selon l'une quelconque des revendications 1 à 3, dans laquelle les électrodes (20, 21) sont des fils, qui sont enroulés sur le noyau (1) en plusieurs pas en forme de spirale ou en forme de rampe.

5. Chambre selon la revendication 4, dans laquelle le noyau (1) comporte, sur la face extérieure, plusieurs cannelures (3, 4) ou rainures parallèles, qui s'étendent en forme de spirale ou en forme de rampe et dans lesquelles sont guidés les fils (20, 21).

6. Chambre selon la revendication 4 ou 5, dans laquelle les fils (20, 21) comportent des extrémités pliées, qui sont insérées dans des forures radiales (12, 13, 14, 15) du noyau (1).

7. Chambre selon la revendication 6, dans laquelle les extrémités pliées sont fixées dans la forure radiale (12, 13, 14, 15) au moyen d'une colle non cytotoxique.

8. Chambre selon l'une quelconque des revendications 1 à 3, dans laquelle les électrodes (20, 21) sont des bandes conductrices disposées sur le noyau (1).

9. Chambre selon la revendication 8, dans laquelle un agent adhésif est disposé entre le noyau (1) et les bandes conductrices.

10. Chambre selon la revendication 1 ou 9, dans laquelle les bandes conductrices comportent une structure interdigitale.

11. Chambre selon l'une quelconque des revendications 1 à 10, dans laquelle le noyau (1) comporte un filetage extérieur (7) au-dessus des électrodes (20, 21) et le bécher (22) comporte, en dessous d'une ouverture (25), un filetage intérieur (26) pour le vissage du filetage extérieur (7).

12. Chambre selon l'une quelconque des revendications 1 à 11, dans laquelle le noyau (1) possède une tête (8) au-dessus des électrodes (20, 21).

13. Chambre selon l'une quelconque des revendications 1 à 12, laquelle comporte un trou borgne (11) axial, qui s'étend depuis l'extrémité supérieure du noyau (1) à peu près jusqu'au niveau des électrodes (20, 21).

14. Chambre selon l'une quelconque des revendications 1 à 13, dans laquelle les électrodes (20, 21) sont reliés par un connecteur enfichable (28) à la face supérieure du noyau (1).

15. Chambre selon l'une quelconque des revendications 1 à 14, dans laquelle les électrodes (20, 21) sont en platine.

16. Chambre selon l'une quelconque des revendications 1 à 15, dans laquelle le noyau (1) est en vitrocéramique.

17. Chambre selon l'une quelconque des revendications 1 à 16, avec un noyau (1) formé par enlèvement de copeaux dans une vitrocéramique usinable par enlèvement de copeaux ou moulé par injection en vitrocéramique.

18. Chambre selon l'une quelconque des revendications 1 à 17, avec un bécher (22) en PC.

19. Chambre selon l'une quelconque des revendications 14 à 18, avec un connecteur enfichable (28) en PEEK.
